# EUROPÄISCHE PATENTSCHRIFT

(11) **EP 1 140 199 B1**
(45) Veröffentlichungstag und Bekanntmachung des Hinweises auf die Patenterteilung: **23.07.2003**
(21) Anmeldenummer: 99962267.3
(22) Anmeldetag: 22.12.1999
(51) Int. Cl.: A61K 47/46, A61P 17/00, A61K 31/17, A61K 38/46

(54) **HYALURONATLYASE ALS PENETRATIONSFÖRDERER IN TOPISCHEN MITTELN**
HYALURONATE LYASE USED FOR PROMOTING PENETRATION IN TOPICAL AGENTS
HYALURONATE LYASE UTILISEE DANS DES MEDICAMENTS TOPIQUES COMME AGENT FACILITANT LEUR PENETRATION

(30) Priorität: 23.12.1998 DE 19860545
(43) Veröffentlichungstag der Anmeldung: 10.10.2001
(73) Patentinhaber: Esparma GmbH, 39171 Osterweddingen (DE)
(72) Erfinder: WOHLRAB, Wolfgang, D-06110 Halle (DE); NEUBERT, Reinhard, D-06120 Halle (DE); HUSCHKA, Christoph, D-06110 Halle (DE); MÜLLER, Peter-Jürgen, Hans-Knöll-Institut, 07745 Jena (DE); OZEGOWSKI, Jörg-Herman, Jena 07745 (DE); KOEGST, Dieter, D-39171 Osterweddingen (DE); FRIES, Gerhard, Esparma GmbH, D-39171 Osterweddingen (DE)
(74) Vertreter: Pfenning, Meinig & Partner
(86) Internationale Anmeldenummer: EP9910337
(87) Internationale Veröffentlichungsnummer: WO00038732

(56) Entgegenhaltungen:
- FR-A- 2 448 903
- GB-A- 1 179 787
- JEDRZEJAS I J ET AL: "Expression and purification of Streptococcus pneumoniae hyaluronate lyase from Escherichia coli." PROTEIN EXPRESSION AND PURIFICATION, (1998 JUN) 13 (1) 83-9. , XP000881665
- GALIKEEV, KH. L.: "Role of hyaluronidase of streptococci in the corpuscular antigen resorption mechanism and its penetration through the mucous barrier of respiratory tracts" ZH. MIKROBIOL., EPIDEMIOL. IMMUNOBIOL. (1967), 44(3), 60-3 , XP002134978

## Beschreibung

Die Erfindung betrifft ein topisch anwendbares Mittel, das Hyaluronatlyasen zur Penetrationsmodulation von einem oder mehreren Wirkstoffen und/oder Arzneistoffen zur kosmetischen und medizinischen Behandlung von Haut, Schleimhaut, Haaren und/oder Nägeln, wie z.B. zur Behandlung von Erkrankungen oder Funktionsstörungen menschlicher oder tierischer Haut, Schleimhaut, Haare und/oder Nägel enthält.

Arzneistoffe und/oder Wirkstoffe werden zusammen mit Hilfsstoffen in Form von flüssigen, halbfesten oder auch festen Formulierungen unterschiedlicher Zusammensetzung zur Behandlung, Prophylaxe und Metaphylaxe von Funktions- und Strukturstörungen der Haut und/oder Schleimhaut eingesetzt. In Abhängigkeit von den physikochemischen Eigenschaften des Wirk- bzw. Arzneistoffes, der verwendeten Grundlage und der darin enthaltenen Hilfsstoffe sowie der Beschaffenheit des zu behandelten Areals der Haut, dringen die Wirkstoffmoleküle über verschiedene Penetrationsmechanismen in die Haut ein, im gegebenen Falle durchdringen sie diese bis zur Resorption durch das Gefäßsystem.

Die Haut ist durch ihre natürliche Barrierefunktion gegenüber der Umwelt gekennzeichnet. Somit kann sie dem Vordringen von äußerlich angewendeten Substanzen und/oder Substanzgemischen entgegenwirken. Die eingesetzten Wirk- und/oder Arzneistoffe bzw. die coapplizierten Hilfsstoffe müssen die Barriere des Stratum corneums überwinden, um in der angestrebten Gewebsschicht (Epidermis, Dermis, bzw. auch in den Follikeln und dem Gefäßsystem) die Wirkung zu entfalten.

Es ist bereits seit langem bekannt, daß durch die Anwesenheit von einem oder mehreren speziellen Hilfsstoffen die Penetration von Wirkstoffen erleichtert werden kann (Penetrationsmodulation). Dabei sind diese Stoffe in der Lage, über unterschiedliche, teilweise nicht vollständig geklärte Wirkungsmechanismen, die Penetration bzw. Permeation von Wirk- und/oder Arzneistoffen zu verändern.

Eine als Enhancereffekt bezeichnete Wirkung besitzen beispielsweise Dimethylsulfoxid, Dimethylacetamid, Dimethylformamid, Derivate des Polyethylenglycol und des Propylenglycol, Polyoxyethylenester, Alkanolamide, Alkanolamine, Alkylamine, N-Alkylpyrrolidone, Diethylenglycolmonoethylether oder Fettsäureglycolester (U.S. 5,912,009), 1-Dodecylazacycloheptan-2-on, Menthol (U.S. Pat. Nr. 4,933,184), pflanzliche Öle (U.S. Pat. 5,229,130) Isopropylmyristat (U.S. Nr. 5,618,555), Eukalyptusöl (U.S. Pat.Nr. 4,440,777), Fettsäureester von Milchsäure (U.S. Pat. Nr. 5,882,676 und 5,952,000) und Lecithin (U.S. No. 4,783,450).

Im U.S. Pat. Nr. 5,296,222 wird ein perkutanes Freisetzungssystem beschrieben, welches Lactamverbindungen (z. B. 1-substituiertes Azacycloheptan-2-on) und Propylenglycol enthält. Das U.S. Pat. Nr. 3,989,816 und 4,316,893 beschreibt ebenfalls die Anwendung von Lactamverbindungen.

In der japanischen Offenlegung Pat. Ser. Nr. 91-251534, werden Pflaster mit erhöhter Penetrationsfähigkeit für Piroxicam durch Anwesenheit von Polyoxyethylenalkylether oder Alkanolamide als Penetrationsbeschleuniger für das basische Arzneimittel Triacetin beschrieben (U.S. No. 5,834,010). Weitere Penetrationsbeschleuniger werden in der U.S. Pat. Nr. 4,755,535 (Azacycloalken) , U. S. Pat. Nr. 4,699,777, (1 -Dodecylazacycloheptan-2-on und Harnstoff zur Aufnahme von Albuterol), U.S. Pat. Nr. 4,820,711 und U.S. Pat, Nr. 4,557,934 (Arzneimittel gelöst in Azone oder Azone-Derivate) vorgeschlagen.

GB1179787 beschreibt die therapeutische Verwendung von Hyaluronatlyase, die nach dem in GB1060513 genannten Verfahren, d.h. aus Hodengewebe, hergestellt wurde.

Das Schutzrecht U.S. Nr. 5,914,322 und Nr. 5,962,433 beschreibt topisch einsetzbare Zubereitungen mit therapeutischer Anwendung bei pathologischen und traumatischen Erscheinungen der Haut, bei der Hyaluronsäure als Transportförderer des Wirkstoffes enthalten ist, Die im U.S. Patent Nr. 5,624,915 und 5,631,242 genannten Kompositionen enthalten Hyaluronsäure, Harnstoff und einen pharmazeutischen Hilfs- und/oder Wirkstoff mit breiter Anwendung gegen verschiedene Haut- und Schleimhauterkrankungen.

Für die Erhöhung der Penetration von hydrophilen Substanzen wurden auch Enzyme als Penetrationsbeschleuniger vorgeschlagen. Die penetrationsfördernde Eigenschaft von Hyaluronidase tierischer Herkunft, insbesondere aus Rinderhoden gewonnen, ist bekannt. Sie wird beispielsweise in Salbenform angewendet [Steinhaus, Christian und Sperandio, J. Amer. Pharmac. Assoc. Sci. Edit. 44, 483 (1955) und Kalentey und Stenszky, Pharmazie 15, 158, (1960)].

Jedrzejas et al. (Protein Expression and Purification 13, 83-89 (1998)) beschreibt die Expression und Aufreinigung von Streptococcus pneumoniae Hyaluronatlyase aus E. coli.

Ein transdermales Freisetzungssystem mit Penetrationsbeschleuniger wird in den französischen Schutzrechten Nr. 2,448,903 und 2,556,218 beschrieben. Die transdermalen Zubereitungen enthalten neben den Arznei- bzw. Wirkstoffen wie z. B. ein Antibiotikum (Tetracyklin) und/oder ein Lokalanesthetikum (Lidocain) und/oder entzündungshemmende Arzneistoffe (Acetylsaliylsäure) und/oder einen emulgierenden oder einen mucolytischen Wirkstoff, Vitamin A sowie als Hilfsstoff Natriumglycerolsterat und/oder mehrere Enzyme als penetrationsfördernden Zusatz. Als Enzyme sind Hyaluronidase, Streptokinase, Streptodornase, Trypsin, Chymotrypsin, alpha-Chymotrypsin, alpha-Amylase, Bromelain, Papain, Desoxyribonuclease, Collagenase und Subtilisin vorgeschlagen worden.

Nachteilig an den genannten Systemen zur Penetrationsförderung ist, daß sie zu Irritationen der Haut führen können. Die Anwesenheit von Proteasen, als auch der als Penetrationsbeschleuniger vorgeschlagenen Lösemittel, führt teilweise zu einer direkten Schädigung der Haut. Bei der Verwendung von Hyaluronidasen boviner Herkunft besteht die Gefahr der Übertragung infektiösen oder antigenen Materials, da sie in der Regel noch große Mengen anderer tierischer Proteine (auch andere Enzymen) enthalten. Das U.S. Patent Nr. 4,152,212 schlägt eine intensive Reinigungsprozedur für ein Rohprodukt einer aus Rinderhoden oder Lebergewebe gewonnenen Hyaluronidase vor.

Nachteilig ist unter anderem am Einsatz der Rinderhodenhyaluronidase als auch anderer Hyaluronidasen tierischer Herkunft, daß das Enzym aufgrund des hohen Fremdproteingehaltes auch nach aufwendigen Reinigungsschritten nur zu einem Produkt mit geringer spezifischer Aktivität führt. Dadurch können pro cm² Haut nur Aktivitäten kleiner als 1.000 IU aufgebracht werden. Inzwischen werden Präparate aus Rinderprodukten für die Anwendung im Menschen wegen des hohen Risikos, die Bovine Spongioforme Encephalopathia (BSE) zu übertragen, kaum noch eingesetzt. Nachteilig ist weiterhin, das die Hyaluronidase durch sulfatierte Glycosaminoglycane, die in der Haut und generell in der extrazellulären Matrix vorkommen, stark gehemmt wird. Dadurch wird die Aktivität des Enzyms in der Haut bei der Anwendung als Penetrationsförderer wesentlich verschlechtert.

Ausgehend hiervon ist es die Aufgabe der vorliegenden Erfindung, ein Mittel zur topischen Anwendung anzugeben, mit dem eine effektive Penetrationspromotion der in dem Mittel enthaltenden Wirk- bzw. Arzneistoffe ermöglicht. Das Mittel soll bevorzugt zur therapeutischen Behandlung und/oder Funktionssteuerung der Haut bzw. Schleimheit einsetzbar sein.

Die Aufgabe wird durch die kennzeichnenden Merkmale des Patentanspruches 1 gelöst. Die Unteransprüche zeigen vorteilhafte Weiterbildungen auf.

Erfindungsgemäß wird somit die Penetration von Arzneistoffen deutlich verbessert, wenn neben mindestens einem oder mehreren Wirk- bzw. Arzneistoffen, vorzugsweise hydrophile Wirk- bzw. Arzneistoffe, ein Enzym vom Typ einer Hyaluronatlyase (E.C. 4.2.2.1) als Penetrationsverstärker in Konzentrationen von 100 IU bis 100.000 IU pro g Formulierung, bevorzugt in Konzentrationen von 500 IU bis 5.000 IU pro g Formulierung vorhanden ist und die Formulierungen topisch angewendet werden.

Allgemein unterscheidet man drei unterschiedlich wirkende hyaluronsäurespaltende Enzymtypen (J. Ludowieg: The Mechanism of Hyaluronidases, JBC 236, 333-339, (1961)]. Es sind einmal die Endohydrolasen, welche die β-(1-3)Bindungen hydrolytisch spalten. Zu ihnen gehören die Mehrzahl der Hyaluronidasen aus höheren Organismen, beispielsweise die Hyaluronidase aus Rinderhoden. Diese Hyaluronat-Glycanhydrolasen (Enzymklasse: E.C. 3.2.1.35/36) spalten neben den Bindungen der Hyaluronsäure in einem begrenzten Maße auch andere Glycosaminoglycane. Einen weiteren Typ stellt die Endo-ß-Hyaluronidase aus dem Blutegel dar, welche die β-(1-4)-Bindung hochspezifisch spaltet. Der dritte Enzymtyp, der erfindungsgemäß verwendet wird, ist die Hyaluronatlyase (Enzymklasse: EC 4.2.2.1). Diese spaltet die Hyaluronsäure in der β-(1-4)-Bindungen nach einem Eliminierungsmechanismus unter Bildung einer Doppelbindung in (4-5)-Stellung an der Glucuronsäure. Für Lyasen werden in der Literatur Endo- und Exospaltmechanismen angegeben.

Die erfindungsgemäße Hyaluronatlyase wird durch mikrobieller Fermentation gewonnen. Als Mikroorganismus wird in einer Ausführung, ohne dadurch den Schutzumfang einzuengen, ein Mikroorganismus der Gattung Streptococcus, bevorzugt ein Streptococcus der serologischen Gruppe B oder C, insbesondere der Art Streptococcus agalactiae eingesetzt.

Ohne die Erfindung im Bezug auf die Herkunft des Enzyms auf einen einzigen Mikroorganismus einzuschränken, soll an Hand des aus Streptococcus agalactiae gewonnen Enzyms die Erfindung beispielhaft beschrieben werden. Das aus den Kulturflüssigkeiten dieses Mikroorganismus hergestellte Enzym besitzt einen Isoelektrischen Punkt von IP = 8,6 und eine Molmasse im Bereich von 116 kD und wirkt nach einem Endo-Spaltmechanismus. Im Verlaufe der Reinigung und Konzentrierung des Enzyms aus dem Kulturfiltrat der Submersfermentation wird in einer weiteren Ausführungsform ein Enzymtrockenpräparat hergestellt und als Trockenpräparat mit einer Aktivität von etwa 10.000 bis 400.000 IU/mg Trockenmasse in die Formulierung eingebracht.

In einer anderen Ausführung wird die Hyaluronatlyase als wäßrige gepufferte Lösung, gegebenenfalls unter Zusatz von Stabilisatoren mit Konzentrationen zwischen 10.000 IU/ml bis 2.000.000 IU/ml in die Formulierung eingebracht.

Die galenische Formulierung kann in Form einer Paste, Salbe, Creme, Emulsion, Gel oder Stiftmasse, bevorzugt als O/W-Emulsion vorliegen. In den Formulierungen können pharmazeutische Träger und/oder hydrophile und/oder lipophile Wirkstoffe und/oder Hilfsstoffe vorhanden sein. Die Hyaluronatlyase und/oder die Wirkstoffe und/oder Hilfsstoffe können in kolloidalen Trägersystemen, vorzugsweise Nanopartikel, Liposomen oder Mikroemulsionen eingearbeitet vorliegen. Als hydrophiler Wirkstoff kann neben allen anderen hydrophilen Arzneimitteln beispielsweise
8-Methoxypsoralen, Harnstoff, Erythromycin, Cyclopiroxolamin, alle im Konzentrationsbereich von 0,01 bis 0,1 g pro g Formulierung oder neben allen anderen lipophilen Arzneimitteln die lipophilen Wirkstoffe Minocyclin im Konzentrationsbereich zwischen 0,001 bis 0,1 g pro g Formulierung oder Glyceroltrinitrat im Konzentrationsbereich zwischen 0,01 bis 0,5 g pro g Formulierung anwesend sein.

Vorteilhaft an den patentgemäßen Formulierungen ist, daß bei Anwendung keine Irritationen der Haut auch bei mehrstündiger Exposition gegenüber den Formulierungen auftreten, die auf das Enzym Hyaluronatlyase zurückzuführen sind. Durch die hohe spezifische Aktivität der Hyaluronatlyase bzw. ihrer hohen Reinheit ist es möglich sehr hohe Aktivitäten pro cm² Hautfläche einzusetzen.

Die Erfindung wird nachfolgend anhand von 10 Beispielen und Penetrationsuntersuchungen näher beschrieben.

### I. Beispiele:

| **1. Wasserhaltige Hydrophile Salbe nach DAB 10 mit Harnstoff (DAB)** | | |
|---|---|---|
| | Harnstoff | 10,00% |
| | Hyaluronatlyase (2500 IU/cm²) | 1,30% |
| | Lanette® N (Emulgierender Cetylstearylalkohol DAB 10) | 9,00% |
| | Paraffinum subliquidum (Dickflüssiges Paraffin) | 10,50% |
| | Vaselinum album (Weißes Vaselin) | 10,50% |
| | Aqua bidestillata ad | 100,00% |
| | | |

| **2. Wasserhaltige Wollwachsalkoholsalbe nach DAB mit Harnstoff (DAB)** | | |
|---|---|---|
| | Harnstoff | 10,00% |
| | Hyaluronatlyase (2500 IU/cm²) | 1,30% |
| | Alcoholes Lanae (Wollwachsalkohole) | 3,00% |
| | Alcohol cetystearylicus (Cetylstearylalkohol) | 0,25% |
| | Vaselinum album (Weißes Vaselin) | 46,75% |
| | Aqua bidestillata ad | 100,00% |
| | | |

| **3. Basiscreme DAC mit Polidocanol (freie Rezeptur)** | | |
|---|---|---|
| | Polidocanol 600 | 5,00% |
| | Hyaluronatlyase (2500 IU/cm²) | 1,30% |
| | Propylenglykol | 3,00% |
| | Aqua bidestillata | 22,00% |
| | Basiscreme DAC ad | 100,00% |
| | | |

| **4. Hydrophile Triamcinolonacetonid-Creme (NRF)** | | |
|---|---|---|
| | Triamcinolonacetonid (mikrofein) | 0,10% |
| | Hyaluronatlyase (2500 IU/cm²) | 1,30% |
| | Mittelkettige Triglyceride | 0,10% |
| | Basiscreme DAC ad | 100,00% |
| | | |

| **5. Hydrophile Hydrocortisonacetat-Creme (NRF)** | | |
|---|---|---|
| | Hydrocortisonacetat | 1,00% |
| | Hyaluronatlyase (2500 IU/cm²) | 1,30% |
| | Nichtionische hydrophile Creme DAB ad | 100,00% |
| | | |

| **6. Harnstoff-Natriumchlorid-Salbe (NRF)** | | |
|---|---|---|
| | Harnstoff | 10,00% |
| | Natriumchlorid | 10,00% |
| | Hyaluronatlyase (2500 IU/cm²) | 1,30% |
| | Gereinigtes Wasser | 30,00% |
| | Wollwachsalkoholsalbe DAB ad | 100,00% |
| | | |

| **7. Hydrophile Harnstoff-Emulsion (NRF)** | | |
|---|---|---|
| | Harnstoff | 10,00% |
| | Milchsäure (90%) | 1,00% |
| | Hyaluronatlyase (2500 IU/cm²) | 1,30% |
| | Natriumlactat-Lösung | 4,00% |
| | Emulsionsgrundlage NRF ad | 100,00% |
| | | |

| **8. Harnstoff-Creme mit Tretinoin (freie Rezeptur)** | | |
|---|---|---|
| | Harnstoff | 12,00% |
| | Tretinoin | 0,03% |
| | Hyaluronatlyase (2500 IU/cm²) | 1,30% |
| | Basiscreme DAC ad | 100,00% |
| | | |

| **9. Hydrophiles MetronidazoI-Gel (NRF)** | | |
|---|---|---|
| | Metronidazol | 0,75% |
| | Propylenglycol | 5,00% |
| | Natriumedetat | 0,10% |
| | Trometamol | 0,25% |
| | Polyacrylsäure | 0,50% |
| | Kaliumsorbat | 0,10% |
| | Hyaluronatlyase (2500 IU/cm²) | 1,30% |
| | Gereinigtes Wasser ad | 100,00% |
| | | |

| **10. Unguentum Oxytetracyclini 1% L/W (SR)** | | |
|---|---|---|
| | Oxytetracyclinum hydrochloricum | 1,00% |
| | Natrium aceticum | 0,36% |
| | Mucilago hydroxyethylcellulosi 8%ASR | 10,00% |
| | Unguentum emulsificans aquosum NSR | 50,00% |
| | Hyaluronatlyase (2500 IU/cm²) | 1,30% |
| | Aqua conservata ASR ad | 100,00% |

### II. Penetration von Harnstoff aus hyaluronatlyasehaltigen Formulierungen im Vergleich zu Standardemulsionen

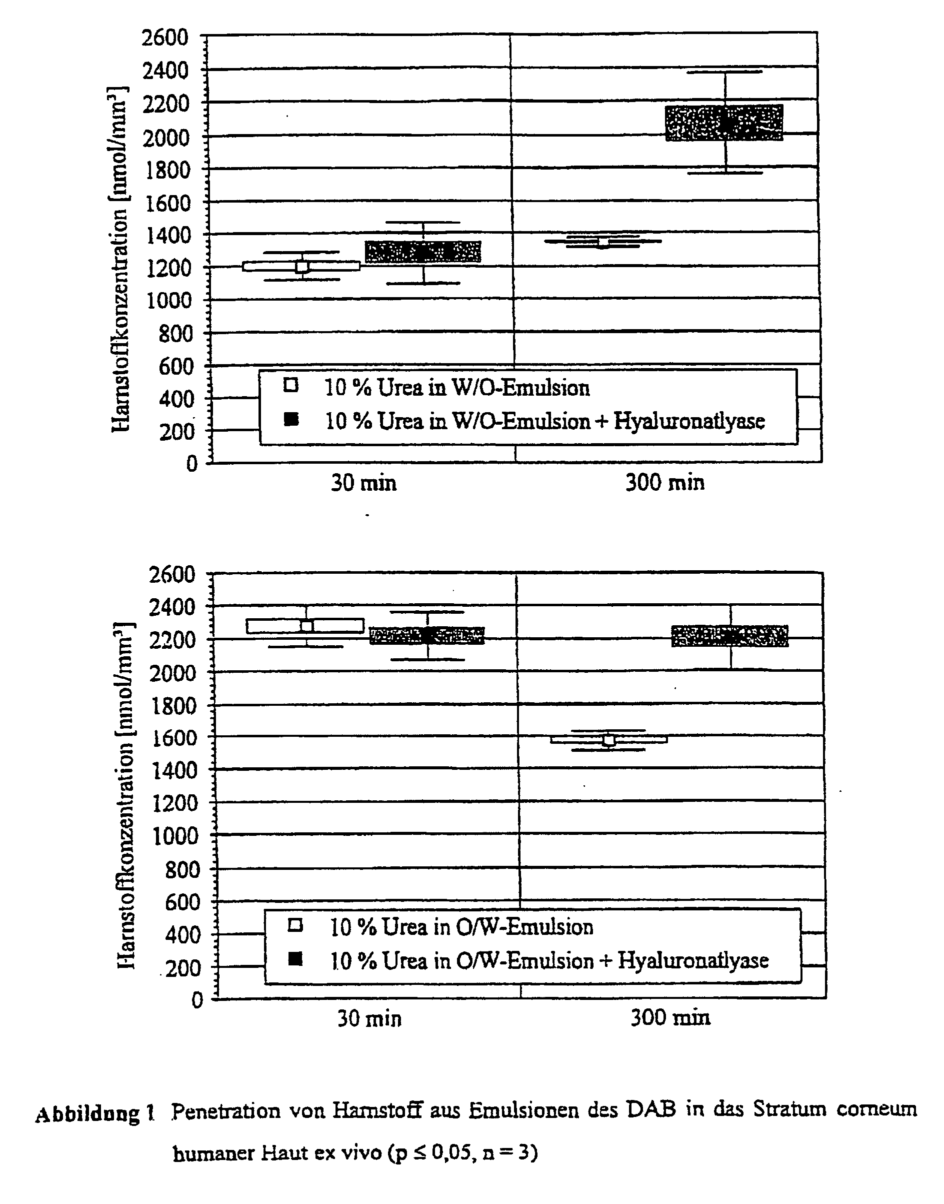

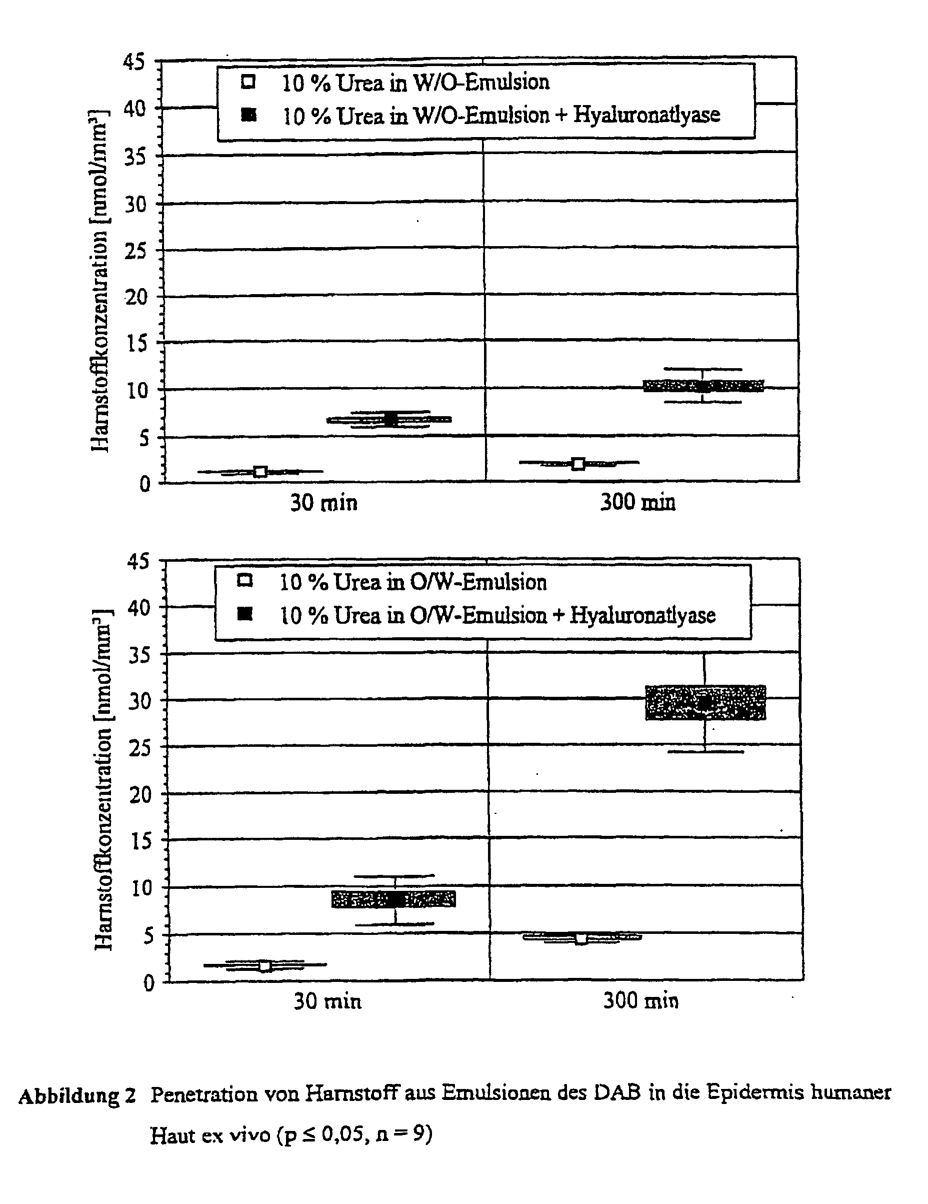

## Patentansprüche

1. Mittel für die topische Anwendung bei der menschlichen oder tierischen Haut und/oder Schleimhaut, enthaltend mindestens einen Penetrationsförderer und mindestens einen Wirkstoff sowie übliche Zusatz- und/oder Hilfsstoffe,
**gekennzeichnet dadurch,**
**daß** es als Penetrationsförderer Hyaluronatlyase in Konzentrationen von 100 IU bis 100000 IU pro g Formulierung enthält und
**daß** die Hyaluronatlyase aus einem Mikroorganismus durch mikrobielle Fermentation erhältlich ist.

2. Mittel nach Anspruch 1, **gekennzeichnet dadurch, daß** die Hyaluronatlyase aus einem Mikroorganismus der Gattung Streptococcus durch mikrobielle Fermentation erhältlich ist.

3. Mittel nach mindestens einem der Ansprüche 1 oder 2, **gekennzeichnet dadurch, daß** die Hyaluronatlyase aus einem Streptococcus der Art Streptococcus agalactiae durch mikrobielle Fermentation erhältlich ist.

4. Mittel nach mindestens einem der Ansprüche 1 bis 3, **gekennzeichnet dadurch, daß** die Hyaluronatlyase einen isoelektrischen Punkt im Bereich von IP = 8,6, eine Molmasse im Bereich von 116 kD aufweist.

5. Mittel nach Anspruch 1, **gekennzeichnet dadurch, daß** die Hyaluronatlyasekonzentration im Bereich von 500 IU bis 5000 IU liegt.

6. Mittel nach mindestens einem der Ansprüche 1 bis 5, **gekennzeichnet dadurch, daß** die Hyaluronatlyase in Form eines gegebenenfalls unter Zusatz von einem oder mehreren Stabilisatoren durch Wasserentzug, insbesondere durch Lyophilisation erhältlichen Trockenproduktes mit einer Aktivität von etwa 10000 bis 400000 IU/mg in der Formulierung eingebracht worden ist.

7. Mittel nach mindestens einem der Ansprüche 1 bis 5, **gekennzeichnet dadurch, daß** die Hyaluronatlyase in Form einer wäßrigen gepufferten Lösung, gegebenenfalls unter Zusatz von Stabilisatoren, mit Konzentrationen von 10000 bis 2.000.000 IU/ml in dieser Form in die Formulierung eingebracht worden ist.

8. Mittel nach mindestens einem der Ansprüche 1 bis 7, **gekennzeichnet dadurch, daß** es als Paste, Salbe, Creme, Emulsion, Gel, Stiftmasse, bevorzugt als O/W-Emulsion vorliegt.

9. Mittel nach mindestens einem der Ansprüche 1 bis 8, **gekennzeichnet dadurch, daß** in den Formulierungen pharmazeutische Träger und/oder hydrophile und/oder lipophile Wirkstoffe und/oder Hilfsstoffe enthalten sind.

10. Mittel nach mindestens einem der Ansprüche 1 bis 9, **gekennzeichnet dadurch, daß** die Hyaluronatlyase und/oder die Wirkstoffe und/oder die Hilfsstoffe in kolloidale Trägersysteme vorzugsweise in Nanopartikel, Liposomen oder Mikroemulsionen eingearbeitet vorliegen.

11. Mittel nach mindestens einem der Ansprüche l bis 10, **gekennzeichnet dadurch, daß** als hydrophiler Wirkstoff Harnstoff im Konzentrationsbereich von 0,01 bis 0,4 g Harnstoff pro g enthalten.

12. Mittel nach mindestens einem der Ansprüche 1 bis 10, **gekennzeichnet dadurch, daß** als lipophiler Wirkstoff 8-Methoxypsoralen im Konzentrationsbereich von 0,001 bis 0,1 g 8-Methoxypsoralen pro g Formulierung enthalten ist.

13. Mittel nach mindestens einem der Ansprüche 1 bis 10, **gekennzeichnet dadurch, daß** als hydrophiler Wirkstoff Erythromycin im Konzentrationsbereich von 0,001 bis 0,1 g Erythromycin pro g Formulierung enthalten ist.

14. Mittel nach mindestens einem der Ansprüche 1 bis 10, **gekennzeichnet dadurch, daß** als lipophiler Wirkstoff Minocyclin im Konzentrationsbereich von 0,001 bis 0,1 g Minocychn pro g Formulierung enthalten ist.

15. Mittel nach mindestens einem der Ansprüche 1 bis 10, **gekennzeichnet dadurch, daß** als hydrophiler Wirkstoff Ciclopiroxolamin im Konzentrationsbereich von 0,001 bis 0,1 g Ciclopiroxolamin pro g Formulierung enthalten ist.

16. Mittel nach mindestens einem der Ansprüche 1 bis 10, **gekennzeichnet dadurch, daß** beispielsweise als lipophiler Wirkstoff Glyceroltrinitrat im Konzentrationsbereich von 0,01 bis 0,5 g Glyceroltrinitrat pro g Formulierung enthalten ist.

## Claims

1. Agent for topical application on human or animal skin and/or mucosa, containing at least one penetration enhancer and at least one active substance plus conventional additives and/or auxiliary substances,
**characterised in that**
it contains as penetration enhancer hyaluronate lyase in concentrations between 100 IU and 100,000 IU per g formulation; and
the hyaluronate lyase is obtainable from a micro-organism by microbial fermentation.

2. Agent according to Claim 1, **characterised in that** the hyaluronate lyase is obtainable from a micro-organism of the type streptococcus through microbial fermentation.

3. Agent according to at least one of Claims 1 or 2, **characterised in that** the hyaluronate lyase is obtainable from a streptococcus of the type streptococcus agalactiae through microbial fermentation.

4. Agent according to at least one of Claims 1 to 3, **characterised in that** the hyaluronate lyase has an isoelectric point in the region of IP = 8.6, a molar mass in the region of 116 kD.

5. Agent according to Claim 1, **characterised in that** the hyaluronate lyase concentration is in the range between 500 lU and 5,000 IU.

6. Agent according to at least one of Claims 1 to 5, **characterised in that** the hyaluronate lyase has been introduced in the formulation in the form of a dry product which has an activity of approximately 10,000 to 400,000 lU/mg and, if appropriate, with an addition of one or more stabilisers through dehydration, in particular by lyophilisation.

7. Agent according to at least one of Claims 1 to 5,
**characterised in that** the hyaluronate lyase has been introduced into the formulation in the form of an aqueous buffered solution, if appropriate with an addition of stabilisers, with concentrations between 10,000 and 2,000.000 lU/mg in this form.

8. Agent according to at least one of Claims 1 to 7, **characterised in that** it is present as a paste, ointment, cream, emulsion, gel, stick, preferably as an o/w emulsion.

9. Agent according to at least one of Claims 1 to 8, **characterised in that** pharmaceutical carriers and/or hydrophilic and/or lipophilic active substances and/or auxiliary substances are contained in the formulations.

10. Agent according to at least one of Claims 1 to 9, **characterised in that** the hyaluronate lyase and/or the active substances and/or the auxiliary substances are present incorporated into colloidal carrier systems, preferably nanoparticles, liposomes or micro-emulsions.

11. Agent according to at least one of Claims 1 to 10, **characterised in that** urea is used as a hydrophilic active substance in the concentration range between 0.01 and 0.4 g urea per g.

12. Agent according to at least one of Claims 1 to 10, **characterised in that** as a lipophilic active substances 8-methoxypsoralen is contained in the concentration range between 0.001 and 0.1 g 8-methoxypsoral per g formulation.

13. Agent according to at least one of Claims 1 to 10, **characterised in that,** as a hydrophilic active substance, erythromycin is contained in the concentration range between 0.001 and 0.1 g erythromycin per g formulation.

14. Agent according to at least one of Claims 1 to 10, **characterised in that,** as a lipophilic active substance, minocyclin is contained in the concentration range between 0.001 and 0.1 g minocyclin per g formulation.

15. Agent according to at least one of Claims 1 to 10, **characterised in that,** as a hydrophilic active substance, ciclopiroxolamine is contained in the concentration range between 0.001 and 0.1 g ciclopiroxolamine per g formulation.

16. Agent according to at least one of Claims 1 to 10, **characterised in that** for example as a lipophilic active substance, glycerol trinitrate is contained in the concentration range between 0.01 and 0.5 g glycerol trinitrate per g formation.

## Revendications

1. Agent destiné à une application topique sur la peau et/ou la muqueuse humaine ou animale, contenant au moins un activateur de pénétration et au moins un principe actif, ainsi que des additifs et/ou auxiliaires usuels,
**caractérisé en ce qu'**il contient en tant qu'activateur de pénétration une hyaluronate lyase dans des concentrations allant de 100 UI à 100 000 UI par g de formulation et
**en ce que** la hyaluronate lyase peut être obtenue à partir d'un microorganisme par fermentation microbienne.

2. Agent selon la revendication 1, **caractérisé en ce que** la hyaluronate lyase peut être obtenue à partir d'un microorganisme de l'espèce Streptococcus (streptocoque) par fermentation microbienne.

3. Agent selon au moins l'une des revendications 1 ou 2, **caractérisé en ce que** la hyaluronate lyase peut être obtenue à partir d'un streptocoque du type *Streptococcus agalactiae* par fermentation microbienne.

4. Agent selon au moins l'une des revendications 1 à 3, **caractérisé en ce que** la hyaluronate lyase présente un point iso-électrique voisin de PI = 8,6, une masse molaire voisine de 116 kD.

5. Agent selon la revendication 1, **caractérisé en ce que** la concentration en hyaluronate lyase est comprise dans la gamme allant de 500 UI à 5 000 UI.

6. Agent selon au moins l'une des revendications 1 à 5, **caractérisé en ce que** la hyaluronate lyase a été incorporée dans la formulation sous la forme d'un produit sec, pouvant être obtenu, éventuellement avec ajout d'un ou de plusieurs stabilisants, par élimination de l'eau, en particulier par lyophilisation, et présentant une activité comprise entre environ 10 000 et 400 000 UI/mg.

7. Agent selon au moins l'une des revendications 1 à 5, **caractérisé en ce que** la hyaluronate lyase a été incorporée dans la formulation sous la forme d'une solution aqueuse tamponnée, éventuellement avec ajout de stabilisants, selon des concentrations comprises entre 10 000 et 2 000 000 UI/ml.

8. Agent selon au moins l'une des revendications 1 à 7, **caractérisé en ce qu'**il se présente sous la forme d'une pâte, d'un onguent, d'une crème, d'une émulsion, d'un gel, d'une masse en bâton, de préférence sous la forme d'une émulsion huile-dans-eau.

9. Agent selon au moins l'une des revendications 1 à 8, **caractérisé en ce que** sont contenus dans la formulation des supports pharmaceutiques et/ou des principes actifs et/ou des auxiliaires hydrophiles et/ou lipophiles.

10. Agent selon au moins l'une des revendications 1 à 9, **caractérisé en ce que** la hyaluronate lyase et/ou les principes actifs et/ou les auxiliaires se présentent incorporés dans des systèmes de support colloïdaux, de préférence dans des nano-particules, des liposomes ou des micro-émulsions.

11. Agent selon au moins l'une des revendications 1 à 10, **caractérisé en ce qu'**il contient, en tant que principe actif hydrophile, de l'urée dans une gamme de concentration comprise entre 0,01 et 0,4 g d'urée par g.

12. Agent selon au moins l'une des revendications 1 à 10, **caractérisé en ce qu'**il contient, en tant que principe actif lipophile, du 8-méthoxypsoralène dans une gamme de concentration comprise entre 0,001 et 0,1 g de 8-méthoxypsoralène par g de formulation.

13. Agent selon au moins l'une des revendications 1 à 10, **caractérisé en ce qu'**il contient, en tant que principe actif hydrophile, de l'érythromycine dans une gamme de concentration comprise entre 0,001 et 0,1 g d'érythromycine par g de formulation.

14. Agent selon au moins l'une des revendications 1 à 10, **caractérisé en ce qu'**il contient, en tant que principe actif lipophile, de la minocycline dans une gamme de concentration comprise entre 0,001 et 0,1 g de minocycline par g de formulation.

15. Agent selon au moins l'une des revendications 1 à 10, **caractérisé en ce qu'**il contient, en tant que principe actif hydrophile, de la ciclopiroxolamine dans une gamme de concentration comprise entre 0,001 et 0,1 g de ciclopiroxolamine par g de formulation.

16. Agent selon au moins l'une des revendications 1 à 10, **caractérisé en ce qu'**il contient, par exemple, en tant que principe actif lipophile, du trinitrate de glycérol dans une gamme de concentration comprise entre 0,01 et 0,5 g de trinitrate de glycérol par g de formulation.
